# EUROPÄISCHE PATENTANMELDUNG

(11) **EP 4 279 058 A1**
(43) Veröffentlichungstag der Anmeldung: **22.11.2023**
(21) Anmeldenummer: 23166298.2
(22) Anmeldetag: 03.04.2023
(51) Int. Cl.: A61K 8/20, A61K 8/44, A61K 8/46, A61Q 19/10, B01F 23/40

(54) **HERSTELLUNG EINER KOSMETISCHEN REINIGUNGSZUSAMMENSETZUNG**

(30) Priorität: 16.05.2022 DE 102022204750
(71) Anmelder: Beiersdorf AG, 20253 Hamburg (DE)
(72) Erfinder: Koop, Eefje, 22850 Norderstedt (DE); Zia, Caroline, 22767 Hamburg (DE)

(57) **Zusammenfassung**

Beschrieben wird ein mobiles Herstellungsverfahren von Reinigungszubereitungen ausgehend von einer vorzugsweise in Kartuschen bereitgestellten Basislösung bestimmter Viskosität, aufweisend anionisches Tensid, Cocamidopropyl Betaine und Natriumchlorid.

## Beschreibung

Kosmetische Produkte dienen im Allgemeinen nicht nur dazu schön und attraktiv auszusehen, sondern sie tragen mit ihrer Wirkung entscheidend zu einem gesteigerten Selbstwertgefühl und zum Wohlbefinden der Menschen bei. Dementsprechend werden die verschiedensten kosmetischen Produkte zur täglichen Reinigung und Pflege der menschlichen Haut eingesetzt.

Die tägliche Körperreinigung mit Duschgelen dient vornehmlich dazu Schmutz, Schweißreste, Fette, abgelagerte Schmutzpartikel und abgestorbene Hautreste effektiv von der Haut zu entfernen. Duschgele enthalten in der Regel anionische Tenside, welche eine besonders effektive Reinigung ermöglichen. Weiterhin enthalten derartige Reinigungsprodukte oftmals zusätzlich weitere Tenside gewählt aus der Gruppe der nichtionischen und amphoteren Tenside. Neben den Tensiden umfassen die Produkte mindestens Viskositätsregulierer sowie Parfümstoffe. Duschgele weisen eine Viskosität im Bereich von 2500 bis 6500 mPa·s auf. Bei Duschgelen mit 3100 bis 6000 mPa·s wird gewährleistet, dass sich die Produkte leicht auf der Haut verteilen lassen, ohne zu leicht aus der Flasche herauszulaufen.

Hersteller von kosmetischen Reinigungszubereitungen besitzen in der Regel industrielle Großanlagen, um die Produkte kosteneffizient und schnell in Packmittel abfüllen zu können. So wird eine hohe Stückzahl gewährleistet. Die Produktion im Batch-Verfahren umfasst in der Regel ein mechanisches Rührwerk zum effizienten Vermischen der Inhaltstoffe.

Um Produkt herzustellen, welches eine ansprechende Viskosität im oben genannten Bereich haben, gibt es im Prinzip zwei Ansätze. Dieses ergibt sich aus dem Punkt, dass zumeist die Viskosität durch die verdickende Wirkung von Cocamidopropylbetaine in Kombination mit NaCl erzielt wird.

Folglich basiert ein erster Ansatz darauf, dass zunächst in einem ersten Schritt alle Komponenten inklusive anionische Tenside, Natriumchlorid und Parfümphase aber mit Ausnahme von Cocamidopropyl Betaine in einem Mischer gemischt werden und in einem zweiten Schritt Cocamidopropyl Betaine zugegeben und nochmals durchmischt werden. In einem dritten Schritt erfolgt die Abfüllung in geeignete Packmittel. Entsprechend wir zunächst eine effiziente Durchmischung der Komponenten im ersten Schritt erreicht und eine Einstellung der Viskosität im zweiten Schritt erreicht.

Ein zweiter Ansatz basiert darauf, dass zunächst in einem ersten Schritt alle Komponenten inklusive anionische Tenside, Cocamidopropyl Betaine und Parfümphase aber mit Ausnahme von Natriumchlorid in einem Mischer gemischt werden und in einem zweiten Schritt Natriumchlorid zugegeben und nochmals durchmischt werden. In einem dritten Schritt erfolgt die Abfüllung in geeignete Packmittel. Entsprechend wir zunächst eine effiziente Durchmischung der Komponenten im ersten Schritt erreicht und eine Einstellung der Viskosität im zweiten Schritt erreicht.

In beiden Fällen erfolgen die Mischverfahren mit einem Rührwerk und zumeist unter Vakuumbedingungen, so dass ein Aufschäumen verhindert wird. Aufgeschäumte Duschgele könnten nicht gleichmäßig abgefüllt werden. Ein Rührwerk ist beispielsweise eine Schraube oder Propeller, der im Mischer enthalten ist und physikalisch im Kontakt mit dem Duschgel steht.

Verschiedene Komponenten, insbesondere Parfümstoffe haben die Eigenschaft, die Viskosität des Duschgels signifikant zu beeinflussen. Folglich ist es bei den oben beschriebenen Verfahren eine Voraussetzung, dass alle Inhaltstoffe bekannt und somit auch deren Viskositätseffekte bekannt sind. Nur so können die Mengen an Natriumchlorid und Cocamidopropyl Betaine so gewählt werden, dass sich eine Viskosität im Bereich von 2500 bis 6500 mPa·s einstellt. Entsprechend lassen sich industriel große Mengen an Produkten herstellen.

Verbraucher wünschen sich jedoch im zunehmenden Maße individuell nach ihren Vorgaben hergestellte Duschgele. Dabei ist es erstrebenswert, dass der Verbraucher u.a. die Parfümvarianten, Farbstoffe, Wirkstoffe und weitere Aspekte der Produkte selbst auswählen können.

Weiterhin ist es wünschenswert, die Duschgele nach Wüschen des Verbrauchers direkt im Laden herzustellen. Dabei ist es weiterhin wünschenswert, wenn wiederbenutzbare Packmittel verwendet werden können, die vom Verbraucher nach Benutzung abgespült und mit in den Verkaufsladen genommen werden können. Setzt man dazu Misch- und Abfüllmaschinen am Verkaufsort ein, so gehen damit Hygieneanforderungen einher. So ist es zwingend erforderlich, dass es keine Kontaminationsmöglichkeit zwischen dem vom Verbraucher mitgebrachten Packmittel und der Misch- und Abfüllmaschine sowie den darin genutzten Komponenten des Duschgels gibt. Entsprechend ist das Abfüllen aller Bestandteile des Duschgels in das Packmittel und ein anschließendes Durchmischen der Komponenten mittels eines mechanischen Rührwerks, welches in Kontakt mit den Komponenten beim Mischvorgang steht, ausgeschlossen.

Soll nun eine Möglichkeit geschaffen werden, im Laden ein Duschgel nach den Wünschen der Kunden direkt herzustellen und in wiederverwertbare Flasche abzufüllen, bestehen eine ganze Reihe von Problemen.
a) Da die vom Verbraucher gewünschten variablen Komponenten, insbesondere Parfümstoffe variablen Einfluss auf die Viskosität von Formulierungen haben, ist es nicht mehr möglich das oben beschriebene Herstellverfahren anzuwenden, da unbekannt ist, wieviel der viskositätsaufbauenden Komponente im zweiten Prozessschritt zugesetzt werden muss. Folglich ist es nicht mehr möglich Produkte mit gleichbleibender Viskosität bereitzustellen. Zudem besteht die Gefahr, dass bei zu hoher Salzkonzentration für die individuelle Formulierung es zu einer sogenannten Übersalzung, bei dem es zu einem signifikanten Viskositätsabfall kommt. Die Formulierungen werden in diesem Fall oftmals wasserdünn (Vergleichbare Viskosität, wie Wasser).
b) Alle vom Verbraucher gewünschten Formulierungen müssen bei der Herstellung derart mischbar sein, so dass sie auch ohne Rührwerk, welches in Kontakt mit einer Formulierung treten würde, eine stabile Formulierung ohne Phasentrennung und mit gleichmäßiger Durchmischung ausbilden.
c) Weiterhin sollten Effekte, wie das Schäumen bei Herstellung von den Duschgelen weniger stark auftreten, so dass sie einfacher ohne die Anwendung einer Vakuumatmosphäre herstellbar sind.

Überraschend konnte die vorliegende Erfindung die obenstehenden Probleme und Anforderungen adressieren.

Gegenstand der Erfindung ist ein Verfahren zur Herstellung einer kosmetischen Reinigungszubereitung aufweisend die Schritte
a) Bereitstellen einer Basislösung aufweisend
   i. Mindestens ein anionisches Tensid,
   ii. Cocamidopropyl Betaine und
   iii. Natriumchlorid,
   dadurch gekennzeichnet, dass die Basislösung eine Viskosität im Bereich von 500 bis 3000 mPa·s aufweist,
b) Zugabe von Parfümstoffen, Farbstoffen und/oder ggf. Wirkstoffen zur Basislösung und
c) Mischen der Komponenten der in b) erhaltenen Mischung zur Bereitstellung der kosmetischen Reinigungszubereitung.

Überraschend hat sich gezeigt, dass die Basislösung ermöglicht, insbesondere Parfümstoffe und Wirkstoffe zuzusetzen und nach Mischen eine Viskosität im Bereich von 2500 bis 6500 mPa s, vorteilhaft von 3100 bis 6000 mPa·s zu erhalten. Entsprechend kann nun der Kunde im Laden das Parfüm, die Base und Wirkstoffe selektiv auswählen, welches der gewählten Basislösung dann zugegeben wird und nach Mischen ein Produkt mit gewohnter Viskosität erhalten wird. Weiterhin hat sich überraschend gezeigt, dass aufbauend auf der vorgelegten Basislösung eine besonders effektive, schaumarme Mischung der Komponenten möglich ist, so dass auf den Einsatz von Rührwerken, die in Kontakt mit der Mischung treten, verzichtet werden kann. Die Produkte waren überraschend stabil nach dem Mischen sowie über Monate danach, so dass keine Phasentrennung beobachtet werden konnte.

Durch die universell Einsetzbare Basislösung wurde der Mischaufwand nach Kundenwünschen erheblich reduziert, so dass eine kurze Mischzeit erreicht wird. Insbesondere müssen nicht zu viele Phasen zugegeben werden, so dass eine weniger Komplexe Abfüll- und Mischmaschine verwendet werden kann.

Auch Gegenstand der Erfindung ist somit eine Basislösung für kosmetische Reinigungszubereitungen aufweisend
i. Mindestens ein anionisches Tensid,
ii. Cocamidopropyl Betaine und
iii. Natriumchlorid,
dadurch gekennzeichnet, dass die Basislösung eine Viskosität im Bereich von 500 bis 3000 mPa·s aufweist.

Sollten nachfolgend Gewichtsprozentangaben (Gew.-%) ohne Bezugnahme auf eine bestimmte Zusammensetzung oder spezifische Mischung angegeben werden, so beziehen sich diese Angaben immer auf das Gesamtgewicht der Basislösung. Sollten nachfolgend Verhältnisse von Komponenten/Substanzen/Stoffgruppen offenbart werden, so beziehen sich diese Verhältnisse auf Gewichtsverhältnisse der genannten Komponenten/ Substanzen/Stoffgruppen.

Die Formulierungen "erfindungsgemäß", "erfindungsgemäß vorteilhaft", "vorteilhaft im Sinne der Vorliegenden Erfindung" etc. beziehen sich im Rahmen der vorliegenden Offenbarung immer sowohl auf das erfindungsgemäße Verfahren als auch auf die erfindungsgemäße Basislösung.

Sofern nicht anders angegeben wurden alle Versuche unter Normalbedingungen durchgeführt. Der Begriff "Normalbedingungen" bedeutet 20°C, 1013 hPa und eine relative Luftfeuchtigkeit von 50%.

Werden in dieser Offenbarung Viskositätswerte angegeben, so beziehen sich alle Werte auf eine Messung bei 25°C in einer 150 ml Weithalsflasche (VWR Nr.: 807-001) mittels Rheomat R 123 von der Firma proRheo. Der Rheomat R 123 der Firma proRheo GmbH ist ein Rotationsviskosimeter, d. h. ein Messkörper rotiert in der zu vermessenden Substanz. Es wird die Kraft gemessen, die benötigt wird, um den Messkörper in der Probe mit einer vorgegebenen Drehzahl rotieren zu lassen. Aus diesem Drehmoment, der Drehzahl des Messkörpers und den geometrischen Abmessungen des verwendeten Messsystems wird die Viskosität berechnet. Als Messkörper wird der Messkörper Nr.1 (Artikelnr. 200 0191), geeignet für einen Viskositätsbereich bis 10.000 [mPa·s], Drehzahl Bereich 62,5 min-1, verwendet.

Wird der Begriff Haut verwendet, so bezieht dieser sich bevorzugt auf die menschliche Haut.

Gemäß der Erfindung wird eine Basislösung eingesetzt. Die Basislösung umfasst mindestens ein anionisches Tensid.

Anionische Tenside weisen als funktionelle Gruppen in der Regel Carboxylat-, Phosphat-, Sulfat- oder Sulfonatgruppen auf und werden im International Cosmetic Ingredient Dictionary and Handbook, Thirteenth Edition 2010, (ISBN 1-882621-47-6) unter der Bezeichnung "surfactant " geführt. In wässriger Lösung bilden sie im sauren oder neutralen Milieu negativ geladene organische Ionen.

Erfindungsgemäß vorteilhaft einzusetzenden anionischen Tensiden sind Schwefelsäureester, wie
- Alkylethersulfat, insbesondere Natrium-, Ammonium-, Magnesium-, MIPA-, TIPA-Laurylethersulfat, Natriummyristylethersulfat und die unter der INCI Bezeichnung bekannte Substanz Sodium C12-13-Parethsulfat;
- Alkylsulfate, insbesondere Natrium-, Ammonium- und TEA-Laurylsulfat;
sowie Sulfonsäuren und Salze, wie
- Acyl-isethionate, insbesondere Natrium-/ Ammoniumcocoyl-isethionat;
- Alkylarylsulfonate;
- Alkylsulfonate, insbesondere Natriumcocosmonoglyceridsulfat, Natrium C12-14 Olefinsulfonat, Natriumlaurylsulfoacetat und Magnesium PEG-3 Cocamidsulfat,
- Sulfosuccinate, insbesondere Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat;

Weitere vorteilhaft einzusetzende anionische Tenside sind Acylaminosäuren und deren Salze, wie
- Acylglutamate, insbesondere Natriumacylglutamat, Di-TEA-palmitoylaspartat und Natrium Caprylic/ Capric Glutamat;
- Acylpeptide, insbesondere Palmitoyl-hydrolysiertes Milchprotein, Natrium Cocoyl-hydrolysiertes Soja Protein und Natrium-/ Kalium-Cocoyl-hydrolysiertes Kollagen,
- Acylsarcosinate, insbesondere Myristoyl Sarcosin, TEA-lauroyl Sarcosinat, Natriumlauroylsarcosinat und Natriumcocoylsarkosinat;
- Acyltaurate, insbesondere Natriumlauroyltaurat und Natriummethylcocoyltaurat;
- Acyllactylate, insbesondere Lauroyllactylat, Caproyllactylat;
- Acylalaninate;
- Acylglycinate, insbesondere Natriumcocoylglycinat.

Weitere vorteilhaft einzusetzende anionische Tenside sind die nachfolgend spezifizierten Gruppen der Carbonsäuren und Carbonsäurederivaten:
- Carbonsäuren, insbesondere Laurinsäure, Aluminiumstearat, Magnesiumalkanolat und Zinkundecylenat;
- Ester-Carbonsäuren, beispielsweise Calciumstearoyllactylat, Laureth-6-Citrat und Natrium PEG-4-Lauramidcarboxylat;
- Ether-Carbonsäuren, beispielsweise Natriumlaureth-13-Carboxylat und Natrium PEG-6-Cocamide Carboxylat;
sowie Phosphorsäureester und Salze, wie beispielsweise DEA-Oleth-10-Phosphat und Dilaureth-4 Phosphat.

Insgesamt ist es bevorzugt, wenn der Anteil der anionischen Tenside in der Basislösung von 3 Gew.-% bis 10 Gew.-% bevorzugt von 4 Gew.-% bis 8 Gew.-% und insbesondere bevorzugt von 5 Gew.-% bis 7 Gew.-% beträgt, bezogen auf das Gesamtgewicht der Basislösung.

Es ist insbesondere vorteilhaft, wenn die erfindungsgemäße Basislösung ein oder mehrere anionische Tenside gemäß der Formel (I)

RO-(CH₂CH₂O)ₓ-(CH₂-CHR¹O)_{y}-(CH₂CH₂O)_{z}-SO₃⁻ M⁺ (I)

enthält, dadurch gekennzeichnet, dass
R ein linearer oder verzweigter C₈-C₁₈-Alkylrest oder Mischungen verschiedener linearer oder verzweigter C₈-C₁₈-Alkylreste ist;
R¹ ein Methyl, Ethyl oder Gemische davon ist;
M⁺ ein Kation, ausgewählt aus der Gruppe bestehend aus Alkalimetallen, NH₄⁺ und HNR² ₃⁺ ist, wobei R² ausgewählt ist aus der Gruppe bestehend aus linearen oder verzweigten Alkylresten, CH₂CH₂OH und CH₂CH(OH)CH₃;
x eine ganzzahlige Zahl im Bereich von 0 - 3 ist;
y eine ganzzahlige Zahl im Bereich 0 - 10 ist; und
z eine ganzzahlige Zahl im Bereich 0 - 30 ist.

Besonders bevorzugte anionische Tenside der Formel (I) sind gewählt aus der Gruppe Sodium Laureth Sulfate (Natriumlaurylethersulfat), Sodium Myreth Sulfate und/oder Ammonium Laureth Sulfate (Ammoniumlaurylethersulfat).

Enthält die erfindungsgemäße Basislösung ein anionisches Tensid gemäß der Formel (I), vorzugsweise Sodium Laureth Sulfate (Natriumlaurylethersulfat), Sodium Myreth Sulfate und/oder Ammonium Laureth Sulfate (Ammoniumlaurylethersulfat), so ist es bevorzugt, wenn der Anteil dieser anionischen Tenside von 3 Gew.-% bis 10 Gew.-% bevorzugt von 4 Gew.-% bis 8 Gew.-% und insbesondere bevorzugt von 5 Gew.-% bis 7 Gew.-% bezogen auf das Gesamtgewicht der Basislösung beträgt. Insbesondere vorteilhaft sind Sodium Laureth Sulfate (Natriumlaurylethersulfat) und/oder Ammonium Laureth Sulfate in einem Anteil von 3 Gew.-% bis 10 Gew.-% bevorzugt von 4 Gew.-% bis 8 Gew.-% und insbesondere bevorzugt von 5 Gew.-% bis 7 Gew.-% bezogen auf das Gesamtgewicht der Basislösung enthalten.

Weiterhin umfasst die Basislösung Cocamidopropyl Betaine. Vorteilhaft beträgt der Anteil von Cocamidopropyl Betaine in der Basislösung von 1 Gew.-% bis 7 Gew.-% bevorzugt von 2 Gew.-% bis 6 Gew.-% und insbesondere bevorzugt von 2,8 Gew.-% bis 4 Gew.-% bezogen auf das Gesamtgewicht der Basislösung.

Weiterhin umfasst die Basislösung Natriumchlorid. Vorteilhaft beträgt der Anteil der von Natriumchlorid in der Basislösung von 0,01 Gew.-% bis 5 Gew.-% bevorzugt von 0,05 Gew.-% bis 3 Gew.-% und insbesondere bevorzugt von 0,1 Gew.-% bis 2,5 Gew.-% bezogen auf das Gesamtgewicht der Basislösung.

Es ist generell vorteilhaft, wenn das Gewichtsverhältnis von Cocamidopropyl Betaine zu Natriumchlorid von 15:3 bis 1:1, bevorzugt 8:1 bis 1,5:1 und insbesondere bevorzugt von 4,5:1 bis 2:1 in der Basislösung beträgt.

Weiterhin ist es vorteilhaft, wenn die Basislösung Sodium Benzoate und/oder Sodium Salicylate umfasst, da diese zur Konservierung der späteren Formeln genutzt werden können. Sofern Sodium Benzoate enthalten ist, ist es vorteilhaft, wenn der Anteil von Sodium Benzoate von 0,2 bis 1,0 Gew.-%, insbesondere von 0,3 bis 0,7 Gew.-%, bezogen auf das Gesamtgewicht der Basislösung beträgt. Sofern Sodium Salicylate enthalten ist, ist es vorteilhaft, wenn der Anteil von Sodium Salicylate von 0,2 bis 1,0 Gew.-%, insbesondere von 0,3 bis 0,7 Gew.-%, bezogen auf das Gesamtgewicht der Basislösung beträgt.

Neben den oben genannten Tensiden können erfindungsgemäß vorteilhaft weitere Tensiden in der Basislösung enthalten sein. So ist es vorteilhaft, wenn weitere nichtionische Tenside enthalten sind.

Vorteilhaft beträgt der Anteil an nichtionischen Tensiden in der Basislösung von 0,2 bis 2 Gew.-%, bezogen auf das Gesamtgewicht der Basislösung.

Vorteilhaft einzusetzende nichtionische Tenside sind gewählt aus der Gruppe der Alkyl-Glucoside. Insbesondere vorteilhaft sind Coco-Glucoside, Decyl-Glucoside, Caprylyl/Caprylglucoside und/oder Lauryl-Glucoside enthalten.

Ferner ist es erfindungsgemäß möglich neben Cocamidopropyl Betaine weitere amphotere und/oder zwitterionsicheTenside einzusetzen.

Vorteilhaft eingesetzte amphotere und/oder zwitterionische Tenside sind
- Acyl-/dialkylethylendiamin, insbesondere Natriumacylamphoacetat, Dinatriumacylamphodipropionat, Dinatriumalkylamphodiacetat, Dinatrium Cocoamphodiacetat, Natrium Cocoamphomonoacetat, Natriumacylamphohydroxypropylsulfonat, Dinatriumacylamphodiacetat und Natriumacylamphopropionat;
- N-Alkylaminosäuren, insbesondere Aminopropylalkylglutamid, Alkylaminopropionsäure, Natriumalkylimidodipropionat und Lauroamphocarboxyglycinat;
- Weitere Betaine, insbesondere Coco Betaine, und
- Sultaine, insbesondere Lauryl Hydroxy Sultaine.

Insbesondere vorteilhaft sind die unter den INCI-Bezeichnungen bekannten amphotere und/oder zwitterionische Tenside Sodium Cocoamphoacetate, Disodium Cocoamphodiacetate, Sodium Cocoamphopropionate, Disodium Cocoamphodipropionate, Coco Betaine und/oder Lauryl Betaine enthalten.

Vorteilhaft ist die Basislösung dadurch gekennzeichnet, dass die Basislösung eine Viskosität im Bereich von 600 bis 2700 mPa·s, vorteilhaft von 650 bis 2500 mPa·s und insbesondere vorteilhaft von 700 bis 2300 mPa·s aufweist.

Sofern finale Reinigungszubereitungen in Form von Duschgels bereitgestellt werden sollen, welche Opak sind, so ist es ebenfalls erfindungsgemäß möglich, dass die Basislösung bereits ein oder mehrere Opacifier umfasst. Als Opacifier wird insbesondere vorteilhaft Glycol Distearate eingesetzt.

Sofern finale Reinigungszubereitungen in Form von Duschgels bereitgestellt werden sollen, welche stabilisierte Partikel enthalten, so ist es ebenfalls erfindungsgemäß möglich, dass die Basislösung ein oder mehre Polymere zur Stabilisierung von Partikeln umfasst. In diesem Bezug wird "stabilisieren" so verstanden, dass eine gleichmäßige Verteilung der Partikel in der Gesamtzusammensetzung erhalten bleibt, und es zu keine Ablagerungseffekten am Boden des Packmittels kommt. Vorteilhafte Polymere umfassen Cellulose Gum und/oder Cellulosen, welche als unlösliche Fasern vorliegen und so ein stabilisierendes Netzwerk in den Zusammensetzungen ausbilden können. Ein bekanntes Produkt ist Arbalon R-50 Cellulose Liquid von der Fa. Lubrizol. Entsprechend ist es weiter vorteilhaft, wenn die zu stabilisierenden Partikel ebenfalls in der Basislösung enthalten sind. So werden Suspensionsschwierigkeiten im späteren Mischungsprozess umgangen.

Erfindungsgemäß ist es weiterhin vorteilhaft, wenn die Basislösung frei von flüssigen Duftstoffen ist. Flüssigen Duftstoffe sind jede natürlichen oder synthetischen Parfümkomponente, die dem Fachmann auf dem Gebiet der Herstellung von Duftstoffen bekannt ist. Einige Beispiele für Duftstoffkomponenten sind

Geraniol, Geranylacetat, Linalool, Linalylacetat, Tetrahydrolinalool, Citronellol, Citronellylacetat, Dihydromyrcenol, Dihydromyrcenylacetat, Tetrahydromyrcenol, Terpineol, Terpinylacetat, Nopol, Nopylacetat, 2-Phenylethanol, 2-Nopol. Phenylethylacetat, Benzylalkohol, Benzylacetat, Benzylsalicylat, Benzylbenzoat, Styrallylacetat, Amylsalicylat, Dimethylbenzylcarbinol, Trichlormethylphenylcarbinylacetat, p-tert.-Butylcyclohexylacetat, Isononylacetat, Vetiverylacetat, Vetiverol, alpha-n- Amylcinammic Aidehyd, α-Hexylcinammic Aldehyd, 2-Methyl-3- (p-tert.-butylphenyl) propanol, 2-Methyl-3- (p-isopropylphenyl) propanal, 3- (p-tert.-butylphenyl) propanal, Tricyclodecenylacetat, Tricyclodecenylpropionat, 4- (4-Hydroxy-4-methylpentyl) -3-cyclohexencarbaldehyd, 4- (4-Methyl-3-pentenyl) -3-cyclohexencarbaldehyd, 4-Acetoxy-3-pentyltetrahydropyran, Methyldihydrojasmonat, 2-n-Heptylcyclopentanon, 3-Methyl-2-pentylcyclopentanon, n-Decanal, 9-Decenol-1, Phenoxyethylisobutyrat, Phenylacetaldehyddimethylacetal, Phenylacetaldehyddiethylacetal, Geranonitril, Citronellonitril, Cedrylacetat, 3-Isocamphylcyclohexanol, Cedrylmethylether, Isolubin , Heliotropin, Cumarin, Eugenol, Vanillin, Diphenyloxid, Hydroxycitronellal, lonone, Methylionone, Isomethylionone, cis-3-Hexenol und Ester davon, Indan-Moschus-Duftstoffe, Tetralin-Moschus-Duftstoffe, Isochroman-Moschus-Duftstoffe, Ethylenbrassylat und aromatische Nitro-Moschus-Düfte. Weitere bekannte Duftstoffe sind in Arctander, Perfume and Flavour Chemicals (Chemicals), Vol. I und II (1969) und in Arctander, Perfume and Flavour Materials of Natural Origin (1960) beschrieben.

Frei von bedeutet in diesem Zusammenhang, dass keine Duftstoffe zugesetzt werden und so der Anteil von flüssigen Duftstoffe weniger als 0,5 Gew.-%, bevorzugt weniger als 0,4 Gew.-%, bevorzugt weniger als 0,3 Gew.-%, bevorzugt weniger als 0,2 Gew.-%, bevorzugt weniger als 0,1 Gew.-% und insbesondere bevorzugt 0 Gew.-%, bezogen auf das Gesamtgewicht der Basislösung, beträgt. Entsprechend ist es am bevorzugtesten, wenn keine flüssigen Duftstoffe enthalten sind. Duftstoffe sind in diesem Zusammenhang als Parfumöle zu verstehen. Folglich gilt analog: Der Anteil von flüssigen Parfumöle beträgt vorteilhaft weniger als 0,5 Gew.-%, bevorzugt weniger als 0,4 Gew.-%, bevorzugt weniger als 0,3 Gew.-%, bevorzugt weniger als 0,2 Gew.-%, bevorzugt weniger als 0,1 Gew.-% und insbesondere bevorzugt 0 Gew.-%, bezogen auf das Gesamtgewicht der Basislösung, beträgt.

Gemäß dem erfindungsgemäßen Herstellungsverfahren wird zunächst die Basislösung bereitgestellt. Diese kann vorteilhaft in einem Großindustriellen Verfahren bereitgestellt werden. Vorteilhaft erfolgt die Bereitstellung in einer Kartusche, welche vorteilhaft in eine Abfüllmaschine eingesetzt werden kann. Eine derartige Abfüllmaschine kann direkt am Verkaufsort aufgestellt werden. Die Kartusche ist vorteilhaft austauschbar, so dass nach Entleerung gegen eine volle Kartusche mit weiterer Basislösung getauscht werden kann.

Vorteilhaft wird bei der Bereitstellung der Basislösung, die Basislösung in das finale Packmittel gefüllt. Dazu wird die Basislösung vorteilhaft aus einer Kartusche in das Packmittel gepumpt bzw. überführt. Dieser Vorgang erfolgt vorteilhaft in einer nicht stationären Abfüllmaschine. Nicht stationär bedeutet, dass die Abfüllmaschine mobil ist und so an einem Verkaufspunkt am Point of Sale (Verkaufsort) werden kann.

In einem zweiten Schritt wird der bereitgestellten Basislösung Parfümstoffe, Farbstoffen und/oder ggf. Wirkstoffen zugegeben. Vorteilhaft erfolgt die Auswahl der zu zugegeben Substanzen gemäß einer Auswahl durch den Verbraucher bzw. des Bedieners der Abfüllmaschine. Die Parfumstoffe, Farbstoffen und/oder ggf. Wirkstoffen sind vorteilhaft individuell in einer Kartusche abgefüllt, oder diese liegen als Mischung in einer oder mehreren Kartuschen vor. Diese Kartuschen sind vorteilhaft ebenfalls in der Abfüllmaschine enthalten. Vorteilhaft erfolgt die Zugabe von Parfumstoffe, Farbstoffen und/oder ggf. Wirkstoffen über die Kartuschen, wobei die Auswahl der Kartuschen gemäß den Wünschen des Verbrauchers erfolgt bzw. gemäß einer Auswahl des Bedieners der Abfüllmaschine erfolgt. Vorteilhaft erfolgt die Zugabe zur Basislösung ebenfalls in das finale Packmittel. Dazu werden die Parfumstoffe, Farbstoffen und/oder ggf. Wirkstoffen vorteilhaft aus einer oder mehreren Kartuschen in das Packmittel gepumpt bzw. überführt. Vorteilhaft umfasst jede Kartusche ihre eigenes Abgabeventil, so dass es keine Kontamination zwischen den verschiedenen Kartuschen geben kann. Die Parfumstoffe sind insbesondere bevorzugt Parfumöle, welches mindestens zugegeben werden sollte. Vorteilhaft wird mindestens ein Parfümöl im Schritt b) zugeben. Dieser Vorgang erfolgt vorteilhaft in einer nicht stationären Abfüllmaschine.

Vorteilhaft beträgt das Gewichtsverhältnis zwischen Basislösung und der Gesamtmenge an zugegeben Parfümstoffe von 96,5:3,5 bis 99,9:0,1, bevorzugt 97,5:2,5 bis 99,7:0,3 und insbesondere bevorzugt von 98,5:1,5 bis 99,6:0,4.

Der Begriff Parfümstoffe umfasst in diesem Zusammenhang flüssige Duftstoffe, sowie die für die für die flüssigen Duftstoffe nötigen organischen Lösungsmittel und Stabilisatoren. Insbesondere sind Parfumöle enthalten.

Nach Zugabe der Parfumstoffe, Farbstoffen und/oder ggf. Wirkstoffen zur Basislösung erfolgt erfindungsgemäß die Mischung der Komponenten zur Bereitstellung eines kosmetischen Reinigungsprodukts. Vorteilhaft erfolgt die Mischung im finalen Packmittel. Vorteilhaft wird dazu das Packmittel verschlossen. Vorteilhaft erfolgt die Durchmischung mittels mechanischen Schüttelns in Richtung einer oder mehrere Dimensionen. Erfindungsgemäß vorteilhaft wird kein Rührwerk zum Durchmischen eingesetzt. Überraschend hat sich gezeigt, dass sich die gewünschte Viskosität durch Zusammenspiel der Parfumstoffe, Farbstoffen und/oder ggf. Wirkstoffen mit der Basislösung einstellt.

Erfindungsgemäß vorteilhaft die Abfüllung und Durchmischung gemäß der Erfindung in einer Maschine. Jedoch ist es ebenfalls erfindungsgemäß möglich, die Abfüllung und Durchmischung in getrennten Maschinen durchzuführen.

Die Mischzeit und eingestellte Schüttelstärke ist vom Fachmann in Abhängigkeit von der eingesetzten Mischmaschine zu wählen.

Erfindungsgemäß vorteilhaft kann zum Abfüllen und durchmischen eine konventionelle Farbmischmaschine, wie der Typ HA 150-24 vom Hersteller Fast & Fluid Management eingesetzt werden. Diese Maschine umfasst ein Kartuschendispensersystem und eine Schütteleinheit zum Durchmischen. Alternative oder individuell gefertigte Maschine können erfindungsgemäß ebenfalls eingesetzt werden.

Nach der Durchmischung kann der Verbraucher das Packmittel mit dem fertigen Produkt entnehmen. Da dieses vorteilhaft verschlossen durchmischt wurde, ist ein Verschütten oder eine Kontamination beim Mischen und bei der Entnahme aus der Maschine ausgeschossen. Sofern standardisierte Packmittel verwendet werden, ist eine Wiederverwertung des Packmittels erfindungsgemäß möglich und auch bevorzugt.

Entsprechend werden vorteilhaft individualisierte Produkte in recycelbaren Packmittel zugängig gemacht.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

In der nachfolgenden Tabelle werden erfindungsgemäße Beispielrezepturen für Basislösungen aufgeführt.

| **INCI** | **Bsp. 1** | **Bsp. 2** | **Bsp. 3** | **Bsp. 4** | **Bsp. 5** | **Bsp. 6** |
|---|---|---|---|---|---|---|
| Aqua | Ad 100 | Ad 100 | Ad 100 | Ad 100 | Ad 100 | Ad 100 |
| Citric Acid | 1,0 | 1,20 | 1,05 | 0,40 | 0,85 | 0,24 |
| Sodium Benzoate | 0,60 | 0,40 | 0,30 | 1,00 | 0,60 | 0,90 |
| Glycerin | | | 0,05 | | | |
| Prunus Amygdalus Dulcis Oil | | | | 0,30 | 0,01 | |
| Vitis Vinifera Seed Oil | | | | | 0,01 | |
| Sodium Chloride | 0,50 | 2,00 | 1,10 | 0,85 | 0,20 | 0,08 |
| Sodium Myreth Sulfate | | 2,20 | | | | 1,00 |
| Sodium Laureth Sulfate | 6,50 | 7,20 | 4,80 | 5,80 | 6,50 | 4,80 |
| Cocamidopropyl Betaine | 3,50 | 2,40 | 4,00 | 4,20 | 3,50 | 4,00 |
| Decyl Glucoside | 1,80 | | 0,20 | 2,00 | | |
| Tocopherol | 0,01 | | | | 0,01 | |
| Aqua (44,3%) + Coco-Glucoside (29,3%) + Glycol Distearate (19,5%) + Citric Acid (4,5%) + Glycerin (2,4%) | | | 4,00 | 1,20 | 3,0 | |
| Sodium Ascorbyl Phosphate | 0,01 | | | | 0,01 | |
| Sodium Salicylate | 0,45 | | | 0,80 | 0,45 | 0,20 |
| Caprylyl/Capryl Glucoside | 0,80 | 0,20 | 0,90 | | | |
| Mannitol (61,9%) + Microcrystalline Cellulose (35%)+ Helianthus Annuus Seed Oil (2%) + CI 77492 (1%) + Alqin (0,1%) | 0,08 | | | 0,05 | | |
| Aqua (84%) + Glycerin (10%) + Cellulose (2,5%)+ Cellulose Gum (2,5%) + Benzoic Acid (0,6%) + Sodium Benzoate (0,4%) | 4,00 | | | 2,80 | | |
| Aqua (57,3%) + Coco-Glucoside (30%) + Hydrogenated Castor Oil (11,5%) + Citric Acid (1%) + Benzoic Acid (0,2%) | | 0,15 | 0,50 | | 1,0 | |
| | | | | | | |
| Viskosität (mPa s) | 1300 | 2650 | 2950 | 2800 | 2000 | 650 |

| **INCI** | **Bsp. 7** | **Bsp. 8** | **Bsp. 9** | **Bsp. 10** | **Bsp. 11** |
|---|---|---|---|---|---|
| Aqua | Ad 100 | Ad 100 | Ad 100 | Ad 100 | Ad 100 |
| Citric Acid | 0,55 | 0,95 | 0,35 | 0,83 | 0,83 |
| Sodium Benzoate | 0,30 | 0,60 | 0,60 | 0,60 | 0,60 |
| Glycerin | | | 0,5 | | 0,10 |
| Prunus Amygdalus Dulcis Oil | | | | 0,01 | |
| Vitis Vinifera Seed Oil | | 0,20 | | 0,01 | |
| Sodium Chloride | 0,1 | 1,80 | 0,50 | 1,00 | 0,25 |
| Sodium Myreth Sulfate | 1,90 | 3,80 | 1,00 | 3,00 | |
| Sodium Laureth Sulfate | 1,60 | 3,30 | 6,00 | 3,00 | 6,50 |
| Cocamidopropyl Betaine | 1,20 | 2,80 | 2,80 | 3,50 | 3,50 |
| Decyl Glucoside | | | 2,08 | | 1,02 |
| Tocopherol | | | | 0,01 | 0,01 |
| Aqua (44,3%) + Coco-Glucoside (29,3%) + Glycol Distearate (19,5%) + Citric Acid (4,5%) + Glycerin (2,4%) | 1,30 | 0,40 | | 3,0 | |
| Sodium Ascorbyl Phosphate | | | | 0,01 | 0,01 |
| Sodium Salicylate | 0,60 | 0,60 | 0,70 | 0,45 | 0,45 |
| Caprylyl/Capryl Glucoside | | 0,6 | | | |
| Mannitol (61,9%) + Microcrystalline Cellulose (35%)+ Helianthus Annuus Seed Oil (2%) + CI 77492 (1%) + Algin (0,1%) | 0,08 | | | | |
| Aqua (84%) + Glycerin (10%) + Cellulose (2,5%)+ Cellulose Gum (2,5%) + Benzoic Acid (0,6%) + Sodium Benzoate (0,4%) | 3,00 | | | | |
| Aqua (57,3%) + Coco-Glucoside (30%) + Hydrogenated Castor Oil (11,5%) + Citric Acid (1%) + Benzoic Acid (0,2%) | 0,5 | 0,5 | | 1,0 | |
| | | | | | |
| Viskosität (mPa s) | 600 | 2500 | 1250 | 1800 | 1000 |

Den verschiedenen Basislösungen wurden verschiedene Parfum und Wirkstoffe zugesetzt. Dazu wurde die konventionell in den Handelsprodukten Nivea Pflegedusche Lemongrass & Oil und Nivea Creme Sensitive Pflegedusche eingesetzten Parfumstoffe den Basen der Beispiele 1, 5 10 und 11 zugegeben. Das Parfum von Lemongrass & Oil wurde im Gewichtsverhältnis von Basislösung zu Parfum von 110,1:1 eingesetzt. Das Parfum von Nivea Creme Sensitive Pflegedusche wurde im Gewichtsverhältnis von Basislösung zu Parfum von 124:1 eingesetzt.

Anschließend wurden die Produkte mittels Schütteln mit der Hand in mehrere Richtungen für 1 Min. gemischt. Die Parfüms ließen sich überraschend leicht lösen. Zudem ergab sich nur eine begrenzte Schaumbildung.

Nach dem Mischvorgang wiesen alle Mischungen aus Basislösung und Parfum eine Viskosität im Bereich von 3000 bis 5400 mPa s auf. Entsprechend erfolgt die Ausbildung der benötigten Viskosität mittels Zugabe von den Parfumstoffen.

Weitere Versuche in Mischmaschinen mit Kartuschensystemen , wie die bekannten HA 150-24 vom Hersteller Fast & Fluid Management, zeigten, dass über die Dosierung der Parfumstoffe aus den Kartuschen zur Basislösung und anschließendem Mischen (durch Schütteln) der Komponenten ohne mechanisches Rührwerk, eine Vielzahl von Formulierungen mit Viskositäten im Bereich von 3100 bis 6000 mPa s bereitgestellt werden konnten.

Zusätzlich wurden weitere Versuche durchgeführt, bei denen Farbstoffe, wie CI 77492 neben dem Parfum zugesetzt wurde.

Zusätzlich wurden weitere Versuche durchgeführt, bei denen Wirkstoffe, wie Aloe Barbadensis Leaf Juice Powder, Bambusa Vulgaris Shoot Extract und Mentha Piperita Leaf Extract neben dem Parfum zugesetzt wurde.

Zusätzlich wurden weitere Versuche durchgeführt, bei denen Parfümöle der Marke Arabiana Opulence (11478 Trenuit Night Treasure) als Parfumstoff zu den Basislösungen der Beispiele 1, 5, 10 und 11 zugesetzt wurde. Es wurde das erfindungsgemäße Verfahren angewendet und die Einsatzverhältnisse gemäß dem vorstehenden Versuchen gewählt.

## Patentansprüche

1. Verfahren zur Herstellung einer kosmetischen Reinigungszubereitung aufweisend die Schritte
a) Bereitstellen einer Basislösung aufweisend
i. Mindestens ein anionisches Tensid,
ii. Cocamidopropyl Betaine und
iii. Natriumchlorid,
**dadurch gekennzeichnet, dass** die Basislösung eine Viskosität im Bereich von 500 bis 3000 mPa·s aufweist,
b) Zugabe von Parfümstoffen, Farbstoffen und/oder ggf. Wirkstoffen zur Basislösung und
c) Mischen der Komponenten der in b) erhaltenen Mischung zur Bereitstellung der kosmetischen Reinigungszubereitung.

2. Verfahren nach Anspruch 1 **dadurch gekennzeichnet, dass** der Anteil der anionischen Tenside in der Basislösung von 3 Gew.-% bis 10 Gew.-% bevorzugt von 4 Gew.-% bis 8 Gew.-% und insbesondere bevorzugt von 5 Gew.-% bis 7 Gew.-% beträgt, bezogen auf das Gesamtgewicht der Basislösung.

3. Verfahren nach einem der vorhergehenden Ansprüche **dadurch gekennzeichnet, dass** das anionische Tensid gewählt ist aus der Gruppe Sodium Laureth Sulfate, Sodium Myreth Sulfate und/oder Ammonium Laureth Sulfate.

4. Verfahren nach einem der vorhergehenden Ansprüche **dadurch gekennzeichnet, dass** der Anteil von Cocamidopropyl Betaine in der Basislösung von 1 Gew.-% bis 7 Gew.-% bevorzugt von 2 Gew.-% bis 6 Gew.-% und insbesondere bevorzugt von 2,8 Gew.-% bis 4 Gew.-% bezogen auf das Gesamtgewicht der Basislösung, beträgt.

5. Verfahren nach einem der vorhergehenden Ansprüche **dadurch gekennzeichnet, dass** das Gewichtsverhältnis von Cocamidopropyl Betaine zu Natriumchlorid von 15:3 bis 1:1, bevorzugt 8:1 bis 1,5:1 und insbesondere bevorzugt von 4,5:1 bis 2:1 in der Basislösung beträgt.

6. Verfahren nach einem der vorhergehenden Ansprüche **dadurch gekennzeichnet, dass** die Basislösung eine Viskosität im Bereich von 600 bis 2700 mPa s, vorteilhaft von 650 bis 2500 mPa·s und insbesondere vorteilhaft von 700 bis 2300 mPa s aufweist.

7. Verfahren nach einem der vorhergehenden Ansprüche **dadurch gekennzeichnet, dass** der Anteil von flüssigen Duftstoffe und/oder Parfumöle weniger als 0,5 Gew.-%, bevorzugt weniger als 0,4 Gew.-%, bevorzugt weniger als 0,3 Gew.-%, bevorzugt weniger als 0,2 Gew.-%, bevorzugt weniger als 0,1 Gew.-% und insbesondere bevorzugt 0 Gew.-%, bezogen auf das Gesamtgewicht der Basislösung, beträgt.

8. Verfahren nach einem der vorhergehenden Ansprüche, **dadurch gekennzeichnet, dass** die Basislösung in einer Kartusche bereitgestellt wird, welche vorteilhaft in eine nicht stationäre Abfüllmaschine eingesetzt werden kann.

9. Verfahren nach einem der vorhergehenden Ansprüche **dadurch gekennzeichnet, dass** bei der Bereitstellung der Basislösung, die Basislösung in das finale Packmittel gefüllt wird.

10. Verfahren nach Anspruch 8 **dadurch gekennzeichnet, dass** die Basislösung bei der Bereitstellung mittels einer Abfüllmaschine aus einer Kartusche in das finale Packmittel gefüllt wird.

11. Verfahren nach einem der vorhergehenden Ansprüche **dadurch gekennzeichnet, dass** die Parfumstoffe, Farbstoffen und/oder ggf. Wirkstoffen individuell in einer Kartusche abgefüllt sind, oder diese liegen als Mischung in einer oder mehreren Kartuschen vorliegen und aus diesen in das finale Packmittel im Schritt b) gefüllt werden.

12. Verfahren nach Anspruch 11 **dadurch gekennzeichnet, dass** die Auswahl der Parfumstoffe, Farbstoffen und/oder ggf. Wirkstoffen nach Auswahl des Bedieners der Abfüllmaschine erfolgt.

13. Verfahren nach Anspruch 12 **dadurch gekennzeichnet, dass** als Parfumstoffe Parfumöle gewählt werden, wobei mindestens ein Parfümöl im Schritt b) zugegeben wird.

14. Verfahren nach einem der Ansprüche 11 bis 13 **dadurch gekennzeichnet, dass** die Abfüllmaschine eine nicht stationäre Abfüllmaschine ist.

15. Verfahren nach einem der vorhergehenden Ansprüche **dadurch gekennzeichnet, dass** das Gewichtsverhältnis zwischen Basislösung und der Gesamtmenge an zugegeben Parfümstoffe von 96,5:3,5 bis 99,9:0,1, bevorzugt von 97,5:2,5 bis 99,7:0,3 und insbesondere bevorzugt von 98,5:1,5 bis 99,6:0,4 beträgt.

16. Verfahren nach einem vorhergehenden Anspruch **dadurch gekennzeichnet, dass** die Mischung im Schritt c) im finalen Packmittel erfolgt.

17. Verfahren nach einem der vorhergehenden Ansprüche **dadurch gekennzeichnet, dass** die Mischung mittels mechanischen Schüttelns in Richtung einer oder mehrere Dimensionen erfolgt.

18. Verfahren nach einem der vorhergehenden Ansprüche **dadurch gekennzeichnet, dass** kein Rührwerk zum Mischen c) eingesetzt wird.

19. Basislösung für kosmetische Reinigungszubereitungen aufweisend
i. Mindestens ein anionisches Tensid,
ii. Cocamidopropyl Betaine und
iii. Natriumchlorid,
**dadurch gekennzeichnet, dass** die Basislösung eine Viskosität im Bereich von 500 bis 3000 mPa s, vorteilhaft von 600 bis 2700 mPa s, vorteilhaft von 650 bis 2500 mPa·s und insbesondere vorteilhaft von 700 bis 2300 mPa·s aufweist.

20. Basislösung nach Anspruch 19 **dadurch gekennzeichnet, dass** der Anteil von flüssigen Duftstoffe und/oder Parfumöle weniger als 0,5 Gew.-%, bevorzugt weniger als 0,4 Gew.-%, bevorzugt weniger als 0,3 Gew.-%, bevorzugt weniger als 0,2 Gew.-%, bevorzugt weniger als 0,1 Gew.-% und insbesondere bevorzugt 0 Gew.-%, bezogen auf das Gesamtgewicht der Basislösung, beträgt.
